# EUROPÄISCHE PATENTANMELDUNG

(11) **EP 2 174 690 A1**
(43) Veröffentlichungstag der Anmeldung: **14.04.2010**
(21) Anmeldenummer: 09177748.2
(22) Anmeldetag: 14.01.2004
(51) Int. Cl.: A61P 1/08, A61K 31/522, A61K 31/495

(54) **Kombinationspräparat mit Cinnarizin und Dimenhydrinat zur Behandlung von Schwindel**

(30) Priorität: 15.01.2003 DE 10301981
(62) Teilanmeldung aus: 04701888.2
(71) Anmelder: Hennig Arzneimittel GmbH & Co. KG, 65439 Flörsheim (DE)
(72) Erfinder: Schleenhain, Helga, 65439, Flörsheim (DE)
(74) Vertreter: Müller & Schubert

(57) **Zusammenfassung**

Beschrieben wird die Verwendung von Cinnarizin und Dimenhydrinat oder deren physiologisch verträgliche Salze in Kombination zur Behandlung von Schwindel jedweder Genese

## Beschreibung

Die vorliegende Erfindung betrifft die Verwendung von Cinnarizin und Dimenhydrinat oder deren physiologisch verträgliche Salze in Kombination.

Jeder zehnte Patient in der Allgemeinpraxis klagt über Schwindel. Mehr als zwei Millionen Menschen suchen jährlich wegen Gleichgewichtsstörungen und Schwindel ihren Hausarzt auf. Nach Kopfschmerzen ist Vertigo damit das zweithäufigste Krankheitssymptom.

Schwindel wird vom Patienten häufig so beschrieben, als ob er Dreh-, Schwank- oder Liftbewegungen erlebe oder der Boden unter den Füßen schwanke. Andere beschreiben den Schwindel als leichte Bewusstseinstrübung mit Verwirrung und Gangunsicherheit.

Für die Orientierung des Menschen im Raum sind drei Sinnessysteme zuständig:
das optische System,
das vestibuläre System und
das propriozeptive System.

Schwindel wird durch Informationskonflikte dieser drei Sinnessysteme, durch Läsionen in peripheren oder zentralen Gleichgewichtsstrukturen bzw. durch okuläre oder psychogene Störungen ausgelöst. Schwindel kann auch ein frühes Zeichen für eine schwere Erkrankung sein. Ursachen von Schwindel können vaskuläre Erkrankungen, Herz-Kreislauferkrankungen, Stoffwechselerkrankungen und Störungen der Rheologie sein. Aufgrund dieser Vielzahl an möglichen Ursachen ist eine interdisziplinäre Diagnostik erforderlich.

Die Einteilung der verschiedenen vestibulären Schwindelformen wird üblicherweise nach dem Entstehungsort vorgenommen. Man unterscheidet peripher-vestibulären, zentralvestibulären sowie kombiniert zentral/peripher-vestibulären Schwindel.

Aus der Art, wie sich die Schwindelbeschwerden manifestieren, lassen sich bereits Informationen zum Ort der Läsion gewinnen.

Die Differentialdiagnose des Schwindels stützt sich vor allem auf die umfassende Anamnese. Die Anamnese sollte Fragen nach der Art des Schwindels, nach begleitenden vegetativen Störungen, nach schwindelauslösenden Situationen oder Mechanismen, nach Dauer der Schwindelanfälle und nach Grund- oder Begleiterkrankungen enthalten. Ein standardisierter Anamnesebogen, in dem auch der Krankheitsverlauf dokumentiert werden kann, kann hierbei hilfreich sein.

Tests zur Prüfung des vestibulo-okulären Systems beruhen darauf, dass das Gleichgewichtssystem auf einen labyrinthären Reiz mit reflektorischen Augenbewegungen (Nystagmen) antwortet. Augenbewegungen können beim Patienten direkt mittels der Frenzelbrille beobachtet oder mit Hilfe der Elektronystagmographie (ENG) bzw. Video-Okulographie (VOG) aufgezeichnet werden. Auswertbare Parameter hierbei sind die Anzahl der Nystagmusschläge pro Zeiteinheit (Nystagmusfrequenz), die Geschwindigkeit der langsamen Nystagmusphase (GLP, auch: slow phase velocity, SPV) sowie die Nystagmusamplitude. Standardverfahren zur Reizung des Labyrinths sind die kalorische Prüfung mit Wasser oder Luft, mit der jedes Labyrinth einzeln stimuliert und überprüft werden kann, und Drehstuhltests.

Treten bereits ohne Stimulus Nystagmen auf (liegt also ein sog. Spontannystagmus vor), lassen sich aus der Richtung der Nystagmusschläge diagnostische Rückschlüsse ziehen.

Zur Untersuchung des vestibulo-spinalen Systems eignen sich besonders der Romberg-Stehversuch und der Unterberger-Tretversuch. Die Reaktionen des Patienten können direkt beobachtet werden und mittels Posturographie oder Craniocorpographie aufgezeichnet werden.

Je nach Ursache des Schwindels können unterschiedliche therapeutische Ansätze zum Erfolg führen.

Zur medikamentösen Vertigo-Therapie stehen u.a. Antihistaminika, Parasympatholytika, zerebral wirksame Kalziumantagonisten, Benzodiazepine, Neuroleptika, durchblutungsfördernde Medikamente sowie Homöopathika zur Verfügung.

Die Auswahl der optimalen medikamentösen Therapie richtet sich nach der Ursache des Schwindels.

Aufgabe der vorliegenden Erfindung ist es daher, ein therapeutisches System zur Verfügung zu stellen, das alle Arten des Schwindels, also Schwindel jedweder Genese therapierbar macht.

Die Aufgabe wird durch Verwendung von Cinnarizin und Dimenhydrinat in Kombination gelöst.

Ein Gegenstand der vorliegenden Erfindung ist daher die Verwendung von Cinnarizin und Dimenhydrinat oder deren physiologisch verträgliche Salze in Kombination zur Behandlung von Schwindel jedweder Genese.

Ein weiterer Gegenstand der vorliegenden Erfindung ist die Verwendung von Cinnarizin und Dimenhydrinat oder deren physiologisch verträgliche Salze in Kombination zur Herstellung von Arzneimitteln zur Behandlung von Schwindel jedweder Genese.

Gegenstand der vorliegenden Erfindung ist ferner die Verwendung von Cinnarizin und Dimenhydrinat oder deren physiologisch verträgliche Salze in Kombination neben pharmazeutisch verträglichen Hilfs- und/oder Zusatzstoffen zur Herstellung von Arzneimitteln zur Behandlung von Schwindel jedweder Genese

Durch die Erfindung wird also das Problem gelöst, alle Schwindelformen - insbesondere auch die sehr häufig auftretenden nicht eindeutig diagnostizierbaren Schwindelformen - ohne therapeutische Fehlversuche erfolgreich zu behandeln. Durch die erfindungsgemäße Verwendung der Wirkstoffkombination aus Cinnarizin und Dimenhydrinat ist nur eine einzige Medikation notwendig. Dies stellt einen großen Fortschritt in der Therapie des Schwindels dar.

Die einzelnen, erfindungsgemäß in Kombination verwendeten Wirkstoffe sind an sich bekannt.

Cinnarizin (CAS 298-57-7) ist der internationale Freiname (INN) für 1-Benzhydryl-4-trans-cinnamylpiperazin, ist ein Antihistaminikum und Vasodilatator und beispielsweise beschrieben in US-A-2,882,271.

Dimenhydrinat (CAS 523-87-5) ist der internationale Freiname (INN) für das 8-Chlortheophyllin-Salz des Diphenhydramins und ist ein als Antiemetikum und gegen Reisekrankheiten eingesetztes Antihistaminikum und ist beispielsweise in US-A-2,499,058 oder US-A-2,534,813 beschrieben.

Diese Wirkstoffe können erfindungsgemäß auch in Form ihrer physiologisch verträglichen Salze eingesetzt werden. Übliche physiologisch verträgliche anorganische und organische Säuren sind beispielsweise Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure. Weitere geeignete Säuren sind aber auch Theophyllin und dessen Derivate, wie beispielsweise 8-Chlortheophyllin, oder andere Xanthine oder Coffein und dessen Derivate. Weitere verwendbare Säuren sind beispielweise in Fortschritte der Arzneimittelforschung, Bd. 10, Seiten 224-225, Birkhäuser Verlag, Basel und Stuttgart, 1966, und Journal of Pharmaceutical Sciences, Bd. 66, Seiten 1-5 (1977) beschrieben.

Die Säureadditionssalze werden in der Regel in an sich bekannter Weise durch Mischen der freien Base oder deren Lösungen mit der entsprechenden Säure oder deren Lösungen in einem organischen Lösungsmittel, beispielsweise einem niederen Alkohol wie Methanol, Ethanol, n-Propanol oder Isopropanol oder einem niederen Keton wie Aceton, Methylethylketon oder Methyl-isobutylketon oder einem Ether wie Diethylether, Tetrahydrofuran oder Dioxan, erhalten. Zur besseren Kristallabscheidung können auch Mischungen der genannten Lösungsmittel verwendet werden. Darüber hinaus können physiologisch verträgliche wässrige Lösungen von Säureadditionssalzen der erfindungsgemäß verwendeten Wirkstoffe einer wässrigen Säurelösung hergestellt werden.

Die erfindungsgemäß verwendeten Säureadditionssalze der Wirkstoffe können in an sich bekannter Weise, z. B. mit Alkalien oder Ionenaustauschern, in die freie Base überführt werden. Von der freien Base lassen sich durch Umsetzung mit anorganischen oder organischen Säuren, insbesondere solchen, die zur Bildung von therapeutisch verwendbaren Salzen geeignet sind, weitere Salze gewinnen. Diese oder auch andere Salze der neuen Verbindung, wie z. B. das Pikrat, können auch zur Reinigung der freien Base dienen, indem man die freie Base in ein Salz überführt, dieses abtrennt und aus dem Salz wiederum die Base freisetzt.

Gegenstand der vorliegenden Erfindung sind auch Arzneimittel zur oralen, rektalen, subcutanen, intravenösen oder intramuskulären Applikation, die neben üblichen Träger- und Verdünnungsmitteln eine erfindungsgemäße Kombination der Wirkstoffe oder deren Säureadditionssalz als Wirkstoff enthalten.

Die Arzneimittel der Erfindung werden mit den üblichen festen oder flüssigen Trägerstoffen oder Verdünnungsmitteln und den üblicherweise verwendeten pharmazeutischtechnischen Hilfsstoffen entsprechend der gewünschten Applikationsart mit einer geeigneten Dosierung in bekannter Weise hergestellt. Die bevorzugten Zubereitungen bestehen in einer Darreichungsform, die zur oralen Applikation geeignet ist. Solche Darreichungsformen sind beispielsweise Tabletten, Filmtabletten, Dragees, Kapseln, Pillen, Pulver, Lösungen oder Suspensionen oder Depotformen.

Selbstverständlich kommen auch parenterale Zubereitungen wie Injektionslösungen in Betracht. Weiterhin seien als Zubereitungen beispielsweise auch Suppositorien genannt.

Entsprechende Tabletten können beispielsweise durch Mischen des Wirkstoffs mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelantine, Gleitmitteln wie Magnesiumstearat oder Talk und/oder Mitteln zur Erzielung eines Depoteffektes wie Carboxylpolymethylen, Carboxylmethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker, hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit dem erfindungsgemäßen Wirkstoff können zusätzlich geschmacksverbessernde Mittel wie Saccharin, Cyclamat oder Zucker sowie z. B. Aromastoffe wie Vanillin oder Orangenextrakt enthalten. Sie können außerdem Suspendierhilfsstoffe wie Natriumcarboxymethylcellulose oder Konservierungsstoffe wie p-Hydroxybenzoate enthalten. Wirkstoffe enthaltende Kapseln können beispielsweise hergestellt werden, indem man den Wirkstoff mit einem inerten Träger wie Milchzucker oder Sorbit mischt und in Gelatinekapseln einkapselt.

Geeignete Suppositorien lassen sich beispielsweise durch Vermischen mit dafür vorgesehenen Trägermitteln wie Neutralfetten oder Polyäthylenglykol bzw. deren Derivaten herstellen.

Die erfindungsgemäße Verwendung der Wirkstoffkombination zeigt unter anderem die folgenden überraschenden Eigenschaften:
- Mit der erfindungsgemäß verwendeten Wirkstoffkombination kann der Arzt ein weitaus größeres Spektrum von Schwindelpatienten erfolgreich behandeln als mit den Einzelsubstanzen (Ausweitung der therapeutischen Breite, Verbreiterung des Wirkprofils). Cinnarizin als Monosubstanz hat lediglich die Indikation "Schwindel bei diagnostisch abgeklärten Innenohrbeschwerden, d. h. bei peripher-vestibulären Beschwerden".
- Verringerung der Einnahmefrequenz.
- Vereinfachung des Dosierungsschemas.
- Verbesserung der Compliance
- Überraschend ist die Beobachtung, dass bei 2,5fach reduzierter Dosierung der Einzelkomponenten in der erfindungsgemäß verwendeten Wirkstoffkombination im Vergleich zu den entsprechenden Monotherapien die Wirkung statistisch signifikant erhöht war bei nachgewiesener gleichzeitiger Verminderung der Nebenwirkungsinzidenz.
- Durch den Einsatz der fixen Kombination in der erfindungsgemäß verwendeten Wirkstoffkombination lässt sich der synergistische, therapeutische Effekt optimal ausnutzen.

Die folgenden Beispiele erläutern die Erfindung.

### Beispiele:

Wirksamkeitsstudien mit der erfindungsgemäß verwendeten Wirkstoffkombination

Es wurden die unten aufgeführten 12 Klinischen Studien durchgeführt, die alle randomisiert und - mit Ausnahme der experimentellen Studie IX - doppelblind nach den Grundsätzen der "Good Clinical Practice", d. h. nach den jeweils neuesten internationalen Bestimmungen durchgeführt wurden.

### Studie I

Bei dieser Studie handelt es sich um eine multizentrische Studie, in der die erfindungsgemäß verwendete Wirkstoffkombination mit den üblicherweise in der Monotherapie hochdosierten Einzelkomponenten Cinnarizin (50 mg) und Dimenhydrinat (100 mg) sowie mit Plazebo verglichen wurde. Studienzentren waren 3 HNO-Universitätskliniken in Ungarn (Budapest, Pecs, Debrecen). Aufgenommen wurden 246 Patienten, die an peripher-vestibulärem, zentral-vestibulärem oder an der sehr häufig vorkommenden kombinierten Form von peripher-zentral-vestibulärem Schwindel litten. Im Ergebnis erwies sich die erfindungsgemäß verwendete Wirkstoffkombination sowohl gegenüber Plazebo als auch gegenüber den hochdosierten Einzelkomponenten statistisch hochsignifikant (p ≤ 0,001) überlegen.

### Studie II

In diese Plazebo-kontrollierte Studie an der HNO-Klinik der Medizinischen Akademie Magdeburg wurden Patienten mit Schwindel in Folge einer diagnostisch gesicherten vertebrobasilären Insuffizienz aufgenommen. Die erfindungsgemäß verwendete Wirkstoffkombination erwies sich sowohl gegenüber Betahistin als auch gegenüber Plazebo statistisch signifikant (p ≤ 0,01 bzw. p ≤ 0,001) überlegen.

### Studie III

In die Studie an der Hals-Nasen-Ohren-Klinik der Universität Rostock wurden 50 Patienten mit Schwindel infolge einer Neuropathia Vestibularis aufgenommen. Hier wurde die erfindungsgemäß verwendete Wirkstoffkombination mit den Einzelwirkstoffen Cinnarizin (20 mg) und Dimenhydrinat (40 mg) - d. h. mit gleicher Dosierung, wie sie in der erfindungsgemäß verwendeten Wirkstoffkombination vorliegt - verglichen. Im Ergebnis zeigte sich die erfindungsgemäß verwendete Wirkstoffkombination gegenüber beiden Einzelkomponenten statistisch signifikant (p ≤ 0,01) überlegen.

### Studie IV

Auch in Studie IV (3-Zentren: HNO-Klinik der Universität Brünn, Neurologische Universitätsklinik Sofia, HNO-Klinik Pilsen) wurde die erfindungsgemäß verwendete Wirkstoffkombination mit den Einzelkomponenten in "Originalkonzentration" (20 mg Cinnarizin bzw. 40 mg Dimenhydrinat) verglichen. Aufgenommen wurden Patienten, die entweder an zentral-vestibulärem, peripher-vestibulärem oder kombiniert peripher-zentral-vestibulärem Schwindel litten. Auch in dieser Studie zeigte sich die erfindungsgemäß verwendete Wirkstoffkombination gegenüber den Einzelkomponenten statistisch signifikant (p ≤ 0,01) überlegen.

### Studie V

Diese 2-Zentren-Studie wurde an den HNO-Kliniken der Medizinischen Akademie Dresden und der Martin-Luther-Universität Halle durchgeführt. Aufgenommen wurden Patienten, die an peripherem, zentralem oder kombiniert peripher-zentralem Schwindel litten. Vergleichssubstanzen waren die Einzelwirkstoffe in höherer Dosierung, Cinnarizin (50 mg) und Dimenhydrinat (100 mg). Die erfindungsgemäß verwendete Wirkstoffkombination zeigte sich den Einzelsubstanzen gegenüber statistisch hochsignifikant (p < 0,001) überlegen.

### Studie VI

Studie VI wurde in der HNO-Klinik, Pilsen durchgeführt. Eingeschlossen wurden Patienten mit diagnostisch gesichertem Innenohrschwindel. Vergleichssubstanz war in diesem Falle Betahistin. Als Ergebnis zeigte sich eine hochsignifikante (p ≤ 0,001) statistische Überlegenheit der erfindungsgemäß verwendeten Wirkstoffkombination über die bei dieser Indikation als Standard geltende Substanz Betahistin.

### Studie VII

Diese Studie wurde an 3 Studienzentren (HNO-Kliniken in Prag, Pilsen, Budweis) durchgeführt. Hier wurde die erfindungsgemäß verwendete Wirkstoffkombination gegen Betahistin bei Patienten mit akuten Schwindelbeschwerden geprüft. Auch in dieser Studie erwies sich die erfindungsgemäß verwendete Wirkstoffkombination dem Betahistin statistisch signifikant (p ≤ 0,05) überlegen.

### Studie VIII

In Studie VIII wurde in der HNO-Klinik der Universität Olomouc bei Patienten mit diagnostisch gesichertem Morbus Menière die Wirkung der erfindungsgemäß verwendeten Wirkstoffkombination im Vergleich zu Betahistin überprüft. Im Ergebnis zeigte sich zwischen der erfindungsgemäß verwendeten Wirkstoffkombination und dem bei Morbus Menière standardmäßig eingesetzten Betahistin kein statistisch signifikanter Unterschied.

### Studie IX, X

Schließlich wurden noch 2 experimentelle Studien mit der erfindungsgemäß verwendeten Wirkstoffkombination gegen Plazebo bzw. der erfindungsgemäß verwendeten Wirkstoffkombination gegen Betahistin von der "Aerospace Medicine Group" der Johannes-Gutenberg-Universität in Mainz durchgeführt. Hier wurde bei gesunden, freiwilligen Probanden mittels eines exzentrischen Drehstuhls - wie er auch beim Gleichgewichtstraining von Astronauten Einsatz findet - Schwindel durch Rotation bei gleichzeitiger Ausführung von Kopfbewegungen induziert und die Wirkung der erfindungsgemäß verwendeten Wirkstoffkombination mit der von Plazebo bzw. mit der von Betahistin verglichen. In beiden Studien war die Wirkung der erfindungsgemäß verwendeten Wirkstoffkombination auf die Zahl der tolerierten Kopfbewegungen der von Plazebo bzw. Betahistin statistisch signifikant (p ≤ 0,01 bzw. p ≤ 0,001) überlegen.

### Studie XI, XII

Ferner wurden zwei Studien zur Ermittlung des Einflusses der erfindungsgemäß verwendeten Wirkstoffkombination auf die Vigilanz durchgeführt (HNO-Klinik der Universität Würzburg; Institut für Hirnforschung, Bulgarische Akademie der Wissenschaften, Sofia). In beiden Studien zeigte die erfindungsgemäß verwendete Wirkstoffkombination keinen statistisch signifikanten Unterschied auf die Vigilanz sowohl im Vergleich zu Betahistin als auch im Vergleich zu Placebo.

## Patentansprüche

1. Verwendung von Cinnarizin und Dimenhydrinat oder deren physiologisch verträgliche Salze in Kombination zur Behandlung von Schwindel jedweder Genese.

2. Verwendung von Cinnarizin und Dimenhydrinat oder deren physiologisch verträgliche Salze in Kombination zur Herstellung von Arzneimitteln zur Behandlung von Schwindel jedweder Genese.

3. Verwendung von Cinnarizin und Dimenhydrinat oder deren physiologisch verträgliche Salze in Kombination neben pharmazeutisch verträglichen Hilfs- und/oder Zusatzstoffen zur Herstellung von Arzneimitteln zur Behandlung von Schwindel jedweder Genese
